Europäisches Patentamt

European Patent Office

Office européen des brevets

(19)

(11) Numéro de publication: **0 236 218 B1**

## (12) FASCICULE DE BREVET EUROPEEN

(45) Date de publication de fascicule du brevet:
**15.05.91**

(51) Int. Cl.⁵: **A61K 7/48**

(21) Numéro de dépôt: **87400396.5**

(22) Date de dépôt: **23.02.87**

(54) **Préparations cosmétiques contenant un extrait des fruits de Silybum Marianum.**

(30) Priorité: **24.02.86 FR 8602889**

(43) Date de publication de la demande:
**09.09.87 Bulletin 87/37**

(45) Mention de la délivrance du brevet:
**15.05.91 Bulletin 91/20**

(84) Etats contractants désignés:
**BE CH DE FR GB IT LI LU NL SE**

(56) Documents cités:
**DE-A- 2 017 789**

(73) Titulaire: **Bonne, Claude**
**316, avenue d'Occitanie**
**F-34000 Montpellier(FR)**

Titulaire: **Sincholle, Daniel**
**343, avenue de la Trémoulette**
**F-34980 Saint-Clement(FR)**

(72) Inventeur: **Bonne, Claude**
**316, avenue d'Occitanie**
**F-34000 Montpellier(FR)**
Inventeur: **Sincholle, Daniel**
**343, avenue de la Trémoulette**
**F-34980 Saint-Clement(FR)**

(74) Mandataire: **Moncheny, Michel et al**
**c/o Cabinet Lavoix 2 Place d'Estienne d'Or-**
**ves**
**F-75441 Paris Cedex 09(FR)**

## Description

La présente invention concerne une composition cosmétique s' opposant au vieillissement de la peau.

La présente invention concerne plus particulièrement une composition cosmétique contenant à titre de principe actif un extrait des fruits de Silybum Marianum.

L'extrait des fruits de Silybum Marianum L. (Gaertn.) ou Carduus Marianum L. est un mélange riche en flavonolignanes qui contient notamment de la silybine, de la silydianine, de la silandrine, de la silychristine, de la silymonine, des silybinomères provenant de la polymérisation de la silybine, ces constituants étant stabilisés dans l' extrait par divers polyphénols tels que le taxifolol et d ' autres constituants tels que l'alcool déhydrodiconiférylique.

L'extrait des fruits de Silybum Marianum peut être obtenu par extraction des fruits délipidés par un solvant tel que le méthanol, l'éthanol ou l' acétone comme décrit par Wagner et coll. Arzneim. Forsch 1: 8, 688, 1968. L'élimination des lipides peut être effectuée à l'aide de solvants apolaires tels que le tétrachlorure de carbone ou l'éther de pétrole.

La plante et ses principes actifs sont connus pour leur pouvoir protecteur hépatique et utilisés à cette fin en médecine depuis l' Antiquité.

Le document DE-A-2 017 789 décrit l'obtention d'un extrait de chardon-marie à l'aide de polyéthylène glycol comme agent d'extraction ainsi que l'obtention de silymarine à partir de cet extrait. Après dilution avec de l'eau, on sépare un extrait résineux d'une émulsion laiteuse. Il est indiqué que les émulsions obtenues comme sous-produits sont utilisées dans un but cosmétique.

Les demandeurs ont découvert que l' extrait des fruits de Silybum Marianum a une activité anti-radicalaire, et que des compositions topiques contenant de 0,01 à 1% et notamment 0, 1 à 0,5% en poids d ' extrait des fruits de Silybum Marianum peuvent s'opposer aux effets altérants des radicaux libres, responsables en partie du vieillissement cutané.

Les radicaux libres sont produits normalement en quantité très faible au cours de la vie cellulaire. Ils peuvent apparaître sous l'action des radiations de l'U.V. mais aussi du spectre visible. La peau est particulièrement concernée par ce mode de formation en raison de son exposition directe. Cependant ils sont surtout formés lors de réactions d'oxydo-réductions physiologiques. Les radicaux ainsi formés sont normalement éliminés par des systèmes enzymatiques capables de les détruire. Toutefois lorsque ces systèmes sont débordés, soit par formation excessive de radicaux soit par défaut des mécanismes de détoxification, ils entrent en réaction avec les constituants cellulaires et les altèrent.

Les constituants cellulaires qui représentent les cibles privilégiées des radicaux libres ont une grande importance fonctionnelle. Il s'agit en premier lieu des acides gras polyinsaturés entrant dans la constitution des phospholipides dont la membrane cellulaire est formée. Lorsque les radicaux attaquent les acides gras, une désorganisation se propage dans la membrane entravant ses fonctions, notamment dans l'épiderme son rôle de barrière qui assure une bonne hydratation de la peau.

Les protéines et parmi celles-ci les protéines enzymatiques et protéines de structure telles que le collagène et l'élastine constituants essentiels du derme sont aussi une cible pour les radicaux libres. Les acides nucléiques enfin, comme l'ADN, support du code génétique, sont particulièrement sensibles à l'action dénaturante des radicaux libres. Leur altération, même infime mais continue au cours des ans, induit des dommages biochimiques irréparables, responsables pour une part du vieillissement cellulaire.

L'extrait des fruits de Silybum Marianum par son activité anti-radicalaire permet de ralentir le vieillissement de la peau.

La présente invention a en conséquence pour objet une composition cosmétique s'opposant au vieillissement de la peau, caractérisée en ce qu'elle contient à titre de principe actif un extrait des fruits de Silybum Marianum, à une concentration en extrait sec de 0,01 à 1% en poids, et de préférence de 0,1 à 0,5% en poids.

Les Demandeurs ont en outre découvert que l'effet contre le vieillissement de la peau peut être augmenté si l'on ajoute à la composition de 0,01 à 0,5% en poids d'alpha-tocophérol. Il est à noter que l'alpha-tocophérol est déjà présent dans l'extrait des fruits de Silybum Marianum mais à une faible concentration.

Les études suivantes mettent en évidence l'activité anti-radicalaire du principe actif utilisé dans la présente invention.

Dans ces études on a utilisé une solution dans le polyéthylèneglycol 400 de l'extrait éthanolique des fruits délipidés de Silybum Marianum contenant 50 g/l d'extrait sec. Cette solution est désignée ci-après "Extrait SM".

## 1 - Activité anti-radicalaire vis-à-vis du radical anion superoxyde

La formation du radical anion superoxyde ($O_2^-$) est une étape clef dans la production des autres espèces réactives de l'oxygène et des radicaux organiques subséquents.

Le radical superoxyde est produit par un grand nombre de réactions d'oxydo-réductions catalysées par des enzymes telles que la xanthine-oxydase.

L'effet protecteur de l'Extrait SM est démontré vis-à-vis de l'anion superoxyde généré par ce système enzymatique in vitro, en présence de cytochrome C dont on mesure la transformation par spectrophotométrie à 550 nm.

Les résultats sont donnés dans le tableau I.

Ces résultats montrent que l'Extrait SM inhibe la transformation du cytochrome C en piégeant l'anion superoxyde.

### TABLEAU I

Activité anti-radicalaire mesurée selon la méthode modifiée décrite par CRAPO et Coll. 1978 (Methods in Enzymology, 53, 382-393)

| EXTRAIT SM (mg/ml) | INHIBITION DE LA TRANSFORMATION DU CYTOCHROME C (%) |
|---|---|
| 0 | 0 |
| 0,1 | 35 |
| 1 | 51 |
| 10 | 70 |

## 2 - Inhibition de la peroxydation des acides gras polyinsaturés (Bonne et coll. 1985, Annals of Allergy 54, 158-160)

Les plaquettes sanguines représentent un modèle très précieux pour étudier la formation des radicaux libres et les produits susceptibles de les piéger dans le milieu cellulaire. En effet les plaquettes sont particulièrement riches en enzymes qui provoquent la peroxydation des acides gras insaturés. Ces réactions sont initiées par la formation de radicaux et peuvent être aisément mesurées en séparant, par chromatographie, les métabolites formés à partir d'un acide gras marqué au 14C tel que l'acide arachidonique. Celui-ci est métabolisé principalement en thromboxane B2 (TxB2), en acide hydroxyheptadécatriénoïque + malonyldialdéhyde (HHT + MDA) et en acide hydroxy-12-eicosatétraénoïque (12-HETE) dont les précuseurs sont des peroxydes instables. L'extrait SM inhibe la production de ces métabolites en empêchant la formation radicalaire des peroxydes au sein même des cellules, à des concentrations en extrait SM comprises entre 0,10 et 0,25% (calculées en extrait sec).

Les résultats sont donnés dans le tableau II.

## TABLEAU II

Activité anti-peroxydante.

| EXTRAIT SM (mg/ml) | INHIBITION DU METABOLISME OXYDATIF DE L'ACIDE ARACHIDONIQUE (%) |
|---|---|
| 0 | 0 |
| 5 | 3 |
| 10 | 9 |
| 15 | 25 |
| 20 | 44 |
| 25 | 63 |
| 50 | 100 |

3 - Inhibition de la formation de malonyldialdéhyde (MDA) dans la peau

L un des signes du vieillissement cellulaire qui permet d' incriminer les radicaux libres dans le processus de la senescence est l' accumulation de lipofuscines dans les tissus. Ces pigments sont des bases de Schiff résultant de la réaction des amines primaires avec le malonyldialdéhyde (MDA) produit par destruction radicalaire des lipides.

L 'effet protecteur vis-à-vis de ce processus est démontré dans le test suivant (Tableau III) où, appliqué sur la peau de souris atrichis, dilué à 5% dans un excipient cosmétique, l'Extrait SM inhibe la formation de MDA dans le tissu cutané induite par irradiation U. V.

Des souris atrichis pesant 25-30 g sont réparties en lots de 5 animaux et sont traitées comme indiqué dans le tableau. L irradiation U.V. à 300 nm est de 0,3 J/cm2.

Neuf heures après l' arrêt de l' irradiation, des échantillons de peau dorsale sont prélevés, pesés et homogénéisés dans une solution saline tamponnée. L' homogénat est centrifugé et le MDA est dosé dans le surnageant par spectrophotométrie après réaction avec l' acide thiobarbiturique.

TABLEAU III

Activité protectrice vis-à-vis des radicaux induits par irradiation U.V de la peau chez la souris atrichis.

| LOT | ANIMAUX | FORMATION DE MDA (nmol/cm2) |
|-----|---------|------------------------------|
| 1 | Témoins non irradiés | 1 |
| 2 | Irradiés non traités | 60 |
| 3 | Irradiés traités par l'excipient | 58 |
| 4 | Irradiés traités par l'extrait SM | 5 |

Les compositions cosmétiques selon l'invention peuvent en outre contenir éventuellement toutes les substances connues ayant des propriétés bienfaisantes sur la peau, telles que le collagène, l'élastine, l'acide hyaluronique, des lipides composés d'acides gras insaturés, des humectants, des extraits vitaminés, des parfums, des conservateurs, des colorants. Elles peuvent contenir aussi des filtres ou écrans des radiations solaires.

Les compositions cosmétiques selon l'invention peuvent se présenter sous toutes les formes utilisées en cosmétologie, crème ou gel en pots ou en tubes, lait, lotion en flacon de verre ou de plastique et éventuellement en flacon doseur ou encore en ampoules.

L'invention concerne donc en particulier des compositions cosmétiques caractérisées en ce qu'elles se présentent sous forme de crème, gel, lait, lotion pour la peau et tout particulièrement des compositions cosmétiques caractérisées en ce que leurs excipients sont adaptés à l'application sur le visage et sur le cou.

Pour chaque forme cosmétique particulière, on a recours à des excipients appropriés. Ces excipients doivent avoir toutes les qualités habituellement demandées. A titre d'exemples, on peut citer : le stéarate de glycérol, le propylène glycol, la lanoline, la glycérine, l'alcool cétylique, les polyols, les huiles végétales, animales, minérales, les cires, les mouillants, les épaississants, stabilisants et émulsionnants couramment utilisés.

Les différentes formes cosmétiques mentionnées ci-dessus sont obtenues selon les méthodes utilisées dans ce domaine.

La présente invention a en outre pour objet des compositions concentrées destinées à la fabrication des compositions cosmétiques selon l'invention et qui contiennent de 1 à 10% en poids (en extrait sec) d'extrait des fruits de Silybum Marianum et en outre avantageusement de 0,2 à 2% en poids d'alpha-tocophérol. De telles compositions peuvent être incorporées dans des bases appropriées pour compositions cosmétiques.

Comme exemples de telles compositions on peut citer des solutions dans du polyéthylèneglycol d'un extrait alcoolique des fruits délipidés de Silybum Marianum tel que l'Extrait SM mentionné ci-dessus ayant une teneur en extrait sec de 5% en poids. On peut également citer l'extrait désigné SMT comprenant 5% en poids d'extrait de fruits délipidés de Silybum Marianum et 1% en poids d'alpha-tocophérol en solution dans du polyéthylèneglycol 400.

On donnera ci-après des exemples de compositions cosmétiquess (en parties en poids).

1. <u>Crème H/E</u>
. Stéarate de glycérol                              5
. Huile de germe de blé                            10
. Extrait SM ou SMT                        de 2 à 10
. Propylèneglycol                                  3
. Polymère carboxyvinylique                      0,5
. Triéthanolamine                                0,5
. Composition aromatique                         q.s
. Conservateurs                                  q.s
. Eau                                     q.sp.100

2. <u>Lait H/E</u>
. Polyéthoxyetherphosphate d'alcool               2
  oléique
. Alcool cétylique                               0,5
. Lanoline anhydre                               0,5
. Silicone fluide                                  1
. Huile de germe de blé                            2
. Acide stéarique                                  3
. Triéthanolamine                                0,5
. Extrait SM ou SMT                        de 2 à 6
. Silicate de Magnésium et d'Aluminium           0,5
. Composition aromatique                         q.s
. Conservateurs                                  q.s
. Eau                                            q.s

3. <u>Lotion hydro-alcoolique</u>
. Alcool cétylique                               0,5
. Acide stéarique                                  5
. Glycérol                                         2
. Extrait SM ou SMT                                3
. Alginate de Na                                 0,3
. Triéthanolamine                                0,5
. Alcool 94°                                       5

```
.  Eau                                                    83,7

4. Hydrogel alcoolique
.  Alcool 40°                                             95
.  Triéthylamine                                          1,5
.  EDTA                                                   0,05
.  Extrait SM ou SMT                                      5
.  Carbopol 940                                           1,5

5. Hydrogel glycériné
.  Alginate sodique                                       8
.  Glycérol                                               25
.  Solution de borax (15%) dans le glycérol              20
.  Extrait SM ou SMT                                      5
.  Thymol                                                 2
.  Eau                                                    45
```

**Revendications**

1. Composition cosmétique s'opposant au vieillissement de la peau caractérisée en ce qu'elle contient à titre de principe actif un extrait des fruits de Silybum Marianum à une concentration, exprimée en extrait sec, de 0,01 à 1% en poids.

2. Composition selon la revendication 1, caractérisée en ce qu'elle contient de 0,1 à 0,5% en poids, exprimé en extrait sec, d'extrait de fruits de Silybum Marianum.

3. Composition selon la revendication 1 ou la revendication 2, caractérisée en ce qu'elle contient, à titre de principe actif, un extrait alcooliques des fruits de Silybum Marianum.

4. Composition selon la revendication 3, caractérisée en ce qu'elle contient une solution dans du polyéthylèneglycol d'un extrait alcoolique des fruit délipidés de Silybum Marianum.

5. Composition selon l'une quelconque des revendications 1 à 4, caractérisée en ce qu'elle contient de 0,01 à 0,2% en poids d'alpha-tocophérol.

6. Composition concentrée destinée à la fabrication d'une composition cosmétique selon la revendication 1, caractérisée en ce qu'elle contient de 1 à 10% en poids exprimé en extrait sec, d'extrait des fruits de Silybum Marianum.

7. Composition selon la revendication 6, caractérisée en ce qu'elle est constituée par une solution dans du polyéthylèneglycol d'un extrait alcoolique des fruits délipidés de Silybum Marianum.

8. Composition selon la revendication 7, caractérisée en ce qu'elle contient en outre 0,2 à 2% en poids d'alpha-tocophérol.

9. Procédé pour s'opposer au vieillissement de la peau, caractérisé en ce que l'on applique sur la peau au moins un extrait des fruits de Silybum Marianum dans une base cosmétique appropriée.

10. Procédé selon la revendication 9, caractérisé en ce que l'on applique sur la peau une composition cosmétique contenant de 0,01 à 1% en poids, exprimé en extrait sec, d'extrait de fruits de Silybum Marianum.

11. Procédé selon la revendication 10, caractérisé en ce que l'on applique sur la peau une composition qui contient en outre de 0,01 à 0,2% en poids d'alpha-tocophérol.

## Claims

1. Cosmetic composition opposing ageing of the skin, characterised in that it contains as active constituent an extract of the fruits of Silybum Marianum, with a dry extract concentration from 0.01 to 1% by weight.

2. Composition according to claim 1, which contains from 0.1 to 0.5% by weight calculated as dry extract of an extract of the fruits of Silybum Marianum.

3. Composition according to claim 1 or claim 2, characterised in that it contains as active constituent an alcohol extract of the fruits of Silybum Marianum.

4. Composition according to claim 3, characterised in that it contains a polyethelene glycol solution of an alcohol extract of the fruits of Silybum Marianum from which the lipids have been removed.

5. Composition according to any one of claims 1 to 4, which contains from 0.1 to 0.2% by weight of alpha-tocopherol.

6. Concentrated composition intended for the manufacture of a cosmetic composition according to claim 1, charactersied in that it contains from 1 to 10% by weight of an extract of the fruits of Silybum Marianum, calculated as dry extract.

7. Composition according to claim 6, characterised in that it consists of a polyethelyne glycol solution of an alcohol extract of the fruits of Silybum Marianum from which the lipids have been removed.

8. Composition according to claim 7, characterised in that it contains 0.2 to 2% by weight of alpha-tocopheral.

9. Process for opposing ageing of the skin, characterised in that it comprises applying to the skin at least one extract of the fruits of Silybum Marianum in an approriate cosmetic base.

10. Process according to claim 3, characterised in that it comprises applying to the skin a cosmetic composition containing from 0.01 to 1% by weight, calculated as dry extract, of an extract of the fruits of Silybum Marianum.

11. Process according to claim 10, characterised in that it comprises applying to the skin a composition which contains in addition 0.01 to 0.2% by weight of alpha-tocopherol.

## Ansprüche

1. Kosmetische Zusammensetzung gegen das Altern der Haut, dadurch gekennzeichnet, daß sie als Wirkstoff einen Extrakt der Früchte der Silybum Marianum in einer Konzentration von 0,01 bis 1 Gew.-% (ausgedrückt in Trockenextrakt) enthält.

2. Zusammensetzung nach Anspruch 1, dadurch gekennzeichnet, daß sie 0,1 bis 0,5 Gew.-% (ausgedrückt in Trockenextrakt) eines Extrakts der Früchte der Silybum Marianum enthält.

3. Zusammensetzung nach Anspruch 1 oder 2, dadurch gekennzeichnet, daß sie als Wirkstoff einen alkoholischen Extrakt der Früchte der Silybum Marianum enthält.

4. Zusammensetzung nach Anspruch 3, dadurch gekennzeichnet, daß sie eine Lösung eines alkoholischen Extrakts der von Lipid befreiten Früchte der Silybum Marianum in Polyethylenglykol enthält.

5. Zusammensetzung nach einem der Ansprüche 1 bis 4, dadurch gekennzeichnet, daß sie 0,01 bis 0,2 Gew.-% Alpha-Tocopherol enthält.

6. Konzentrierte Zusammensetzung zur Herstellung einer kosmetischen Zusammensetzung nach Anspruch 1, dadurch gekennzeichnet, daß sie 1 bis 10 Gew.-% (ausgedrückt in Trockenextrakt) eines Extrakts der Früchte der Silybum Marianum enthält.

7. Zusammensetzung nach Anspruch 6, dadurch gekennzeichnet, daß sie aus einer Lösung eines alkoholischen Extrakts der von Lipid befreiten Früchte des Silybum Marianum in Polyethylenglykol besteht.

8. Zusammensetzung nach Anspruch 7, dadurch gekennzeichnet, daß sie außerdem 0,2 bis 2 Gew.-% Alpha-Tocopherol enthält.

9. Verfahren zum Entgegenwirken gegen das Altern der Haut, dadurch gekennzeichnet, daß auf die Haut mindestens ein Extrakt der Früchte der Silybum Marianum in einer geeigneten kosmetischen Basis aufgetragen wird.

10. Verfahren nach Anspruch 9, dadurch gekennzeichnet, daß auf die Haut eine kosmetische Zusammensetzung aufgetragen wird, die 0,01 bis 1 Gew.-% (ausgedrückt in Trockenextrakt) Extrakt der Früchte der Silybum Marianum enthält.

11. Verfahren nach Anspruch 10, dadurch gekennzeichnet, daß auf die Haut eine Zusammensetzung aufgetragen wird, die außerdem 0,01 bis 0,2 Gew.-% Alpha-Tocopherol enthält.